# EUROPEAN PATENT APPLICATION

(11) **EP 4 834 406 A2**
(43) Date of publication of application: **04.11.2026**
(21) Application number: 26205541.1
(22) Date of filing: 26.02.2025
(51) Int. Cl.: A61M 25/01

(54) **DUAL-LUMEN CATHETER WITH DETACHMENT TIP**

(30) Priority: 26.02.2024 US 202463558065 P
(62) Document number(s) of the earlier application(s) in accordance with Art. 76 EPC: 25160235.5 / 4 606 412
(71) Applicant: Balt Innovation SAS, 95160 Montmorency (FR)
(72) Inventor: BARTOLOMEO, Brian, Irvine,, 926118 (US); JALGAONKAR, Ujwal, Irvine, 926118 (US)
(74) Representative: Marks & Clerk LLP

(57) **Abstract**

According to an aspect, catheters for embolization of an anatomic vessel of a subject may include a first tube and a second tube, with the first tube defining a first lumen and the second tube defining a second lumen. The second tube may have a second proximal portion, a second distal portion, and a detachment zone therebetween. The first tube may be coupled to the second tube from a proximal portion of the second tube to at least the detachment zone. A distal portion of the second tube may be detachable from the proximal portion of the second tube along the detachment zone.

## Description

### CROSS-REFERENCE TO RELATED APPLICATIONS

This application claims the benefit of priority to U.S. Provisional Patent Application No. 63/558,065, filed on February 26, 2024, the entire contents of which are hereby incorporated herein by reference.

### BACKGROUND

Implantable medical devices can be used to treat a number of diseases and conditions associated with body lumens. For instance, a weakening in the arterial wall can develop into an aneurysm and, ultimately, can lead to internal bleeding and/or other conditions. Implantable medical devices, such as liquid embolics, coils, and stents, can be used to occlude vessels, aneurysms, and other anatomical spaces to treat such potentially dangerous conditions and/or for vessel sacrifice. For example, implantable medical devices can be used to obstruct (e.g., completely or partially) the flow in a blood vessel, such as, a location peripheral or adjacent to an aneurysm or other vascular abnormality. However, the time associated with accurate and stable placement of such implantable medical devices at a treatment site is often critical. Accordingly, there is a need for implant devices that can be quickly and reliably placed for occlusion of vessels, aneurysms, and other anatomical locations.

### SUMMARY

Catheters for embolization of an anatomic vessel of a subject may include a first tube and a second tube, with the first tube defining a first lumen and the second tube defining a second lumen. The second tube may have a second proximal portion, a second distal portion, and a detachment zone therebetween. The first tube may be coupled to the second tube from a proximal portion of the second tube to at least the detachment zone. A distal portion of the second tube may be detachable from the proximal portion of the second tube along the detachment zone. As compared to treating aneurysms using interventional devices delivered using separate catheters, catheters of the present disclosure may facilitate simultaneous or nearly simultaneous delivery of a liquid embolic along with one or more other therapeutic devices. For example, detachment zones of the catheters of the present disclosure may facilitate leaving a distal portion of the second tube at a treatment site in instances in which the distal portion of the second tube becomes entrapped in a cast of liquid embolic.

According to an aspect, a catheter for embolization of an anatomic vessel of a subject may include: a first tube having a first proximal portion, a first distal portion, and a first lumen defined therebetween, the first distal portion defining a first orifice in fluid communication with the first lumen; and a second tube having a second proximal portion, a second distal portion, and a detachment zone therebetween, the second tube defining a second lumen from the second proximal portion to the second distal portion, the second distal portion defining a second orifice in fluid communication with the second lumen, the first tube coupled to the second tube from the second proximal portion to at least the detachment zone, and the second distal portion of the second tube detachable from the second proximal portion along the detachment zone.

In certain implementations, the second distal portion of the second tube may become decoupled from at least a portion of the first tube upon detachment of the second distal portion of the second tube from the second proximal portion along the detachment zone.

In some implementations, the second distal portion of the second tube may be detachable from the second proximal portion in response to an axial stress less than a first failure stress of the first tube coupled to the second tube and less than a second failure stress of the second distal portion of the second tube. As an example, the detachment zone may be an area of weakness in continuous material defining the second lumen of the second tube. As a specific example, at least the second tube may be most brittle along the detachment zone than along any other portion of the second tube. For example, composition of the second tube along the detachment zone may differ from composition along any other portion of the second tube. Further, or instead, the second distal portion of the second tube may be releasably attached to the second proximal portion of the second tube at the detachment zone via an interference fit.

In certain implementations, at least the first lumen may remain operable following detachment of the second distal portion from the second proximal portion along the detachment zone.

In some implementations, the second distal portion of the second tube may extend distally beyond the first distal portion of the first tube. As an example, the second distal portion of the second tube may extend distally beyond the first distal portion of the first tube by greater than about 5 mm and less than about 75 mm.

In certain implementations, the first distal portion of the first tube and the second distal portion of the second tube may be split from one another distal to the detachment zone.

In some implementations, the first tube and the second tube may have the same axial length, the first distal portion of the first tube is detachable with detachment of the second distal portion of the second tube at the detachment zone.

In certain implementations, at least at the detachment zone, the first tube and the second tube collectively form a curvilinear outer circumference having a major dimension and a minor dimension, and the major dimension is greater than the minor dimension. For example, the curvilinear outer circumference may be constant at least from the first proximal portion and the second proximal portion to the detachment zone. As an example, the curvilinear outer circumference may be oval or stadium-shaped.

In some implementations, the first proximal portion of the first tube may define a first access port in fluid communication with the first lumen, the second proximal portion of the second tube defines a second access port in fluid communication with the second lumen, and the first lumen and the second lumen are each fluidically isolated from one another.

In certain implementations, the first lumen may have a first cross-sectional area from the first proximal portion to the first orifice, and the second lumen has a second cross-sectional area from the second proximal portion to the second orifice. At least one of the first cross-sectional area or the second cross-sectional area may be circular. Further, or instead, the first cross-sectional area is constant from the first proximal portion to the first orifice, and the second cross-sectional area is constant from the second proximal portion to the second orifice. As an example, the first cross-sectional area and the second cross-sectional area may be within ± 10 percent of one another.

In some implementations, the first tube may be hydrophobic along the first lumen, and the second tube is hydrophobic along the second lumen.

In certain implementations, the first tube and the second tube may each be at least partially formed of one or more polymers. In some instances, the first tube and the second tube may each be at least partially formed of a respective polymer having Shore D hardness greater than 15D and less than 100D. Further, or instead, the first tube and the second tube are each formed of the same material. Still further, or instead, the respective polymer of at least one of the first tube or the second tube may be loaded with tungsten, barium sulfate, or a combination thereof, at least along the detachment zone. Additionally, or alternatively, at least a portion of each of the first tube and the second tube may include shielding in the one or more polymers proximal to the detachment zone.

In some implementations, the catheter may further include a proximal radiopaque marker proximal and adjacent to the detachment zone of the second tube.

In certain implementations, the catheter may further include a first distal radiopaque marker and a second distal radiopaque marker, wherein the first distal radiopaque marker is adjacent to the first orifice of the first tube, and the second distal radiopaque marker is adjacent to the second orifice of the second tube.

In some implementations, the first lumen and the second lumen may each have a diameter greater than 0.05 mm and less than 0.5 mm.

In certain implementations, the first tube and the second tube may each be directly coupled to one another from the second proximal portion to at least the detachment zone. As an example, the first tube and the second tube may each be welded to one another from the second proximal portion at least the detachment zone.

In some implementations, the catheter may further include intermediate member, wherein the first tube and the second tube are coupled to one another from the second proximal portion to at least the detachment zone via the intermediate member, and Shore D hardness of the intermediate member is different from respective Shore D hardness of each of the first tube and the second tube.

According to another aspect, a system for embolization of an anatomic vessel of a subject may include: a catheter having a proximal region and a distal region, the catheter defining a first lumen and a second lumen from the proximal region to the distal region, at least one section of the distal region defining the second lumen detachable from the proximal region of the catheter at a detachment zone; a coil deliverable to a treatment site via the first lumen; and a liquid embolic deliverable to the treatment site via the second lumen and, at the treatment site, the liquid embolic curable on the at least one section of the distal region defining the second lumen. The liquid embolic may include dimethyl sulfoxide (DMSO).

According to yet another aspect, a method of embolization of an anatomic vessel may include: moving a catheter to a treatment site; delivering a first treatment to the treatment site via a first lumen defined from a proximal region to a distal region of the catheter; delivering a second treatment to the treatment site via a second lumen defined from the proximal region to the distal region of the catheter, the second treatment including curing a liquid embolic; and, with the liquid embolic solidified thereon, detaching at least one section of the distal region defining the second lumen.

In certain implementations, the first treatment may include delivering a coil to the treatment site via the first lumen. For example, the coil may be proximal to the liquid embolic at the treatment site.

In some implementations, delivering the coil to the treatment site via the first lumen may include retracting the coil into the first lumen with the liquid embolic solidified at the treatment site.

In certain implementations, the liquid embolic includes dimethyl sulfoxide (DMSO).

### BRIEF DESCRIPTION OF THE DRAWINGS

FIG. 1A is a schematic representation of a system for embolization of an anatomic vessel of a subject, the system including a catheter, a coil, and a liquid embolic.
FIG. 1B is a schematic representation of an area of detail 1B in FIG. 1A, with the area of detail showing the distal region of the catheter of FIG. 1A.
FIG. 1C is a cross-section of the catheter of FIG. 1A along the cross-section 1C-1C in FIG. 1B.
FIG. 1D is the schematic representation of FIG. 1B shown in use at a treatment site.
FIG 2 is a flowchart of an exemplary method of embolization of an anatomic vessel.
FIG. 3 is a cross-section of a catheter including a first tube and a second tube coupled to one another via an intermediate member.

Like reference symbols in the various drawings indicate like elements.

### DESCRIPTION

Embodiments will now be described with reference to the accompanying figures. The foregoing may, however, be embodied in many different forms and should not be construed as limited to the embodiments set forth herein.

All documents mentioned herein are hereby incorporated by reference in their entirety. References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and, similarly, the term "and" should generally be understood to mean "and/or."

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately," or the like, when accompanying a numerical value, are to be construed as indicating a deviation as would be appreciated by one of ordinary skill in the art to operate satisfactorily for an intended purpose. Ranges of values and/or numeric values are provided herein as examples only, and do not constitute a limitation on the scope of the described embodiments. The use of any and all examples, or exemplary language ("e.g.," "such as," or the like) provided herein, is intended merely to describe the embodiments better and does not pose a limitation on the scope of the embodiments. No language in the specification should be construed as indicating any unclaimed element as essential to the practice of the embodiments.

In the following description, it is understood that terms such as "first," "second," and the like, are words of convenience and are not to be construed as limiting terms. For example, in the description that follows, implants are described as having a first section and a second section. It shall be appreciated that this is for the sake of clear and efficient description and should not be understood to be limiting, unless a contrary intent is explicitly indicated or is made clear from the context. Thus, any one or more of the implants of the present disclosure may have two or more section that are different from one another, as may be useful for achieving rapid and reliable placement of implants for vascular treatment.

As used herein, unless otherwise indicated or made clear from the context, the term "physician" should be understood to include a surgeon or other interventional specialist preparing for and/or performing any one or more of the medical procedures described herein and, more broadly, should be understood to include any medical personnel, such as nurses, assisting a such surgeon or interventional specialist in preparing for or performing any one or more of the medical procedures described herein. Further, as used herein, the term "subject" shall be understood to include any type of mammal, including a human, on which a medical procedure such as, but not limited to a thrombectomy can be performed.

For the sake of convenience and clear description, unless otherwise specified or made clear from the context, the term "liquid embolic" is used herein to refer to both an embolic, glue, or a combination thereof and shall be understood to refer to the state of the material deliverable to a treatment site via a catheter and to the state of the material as it solidifies at the treatment site. Thus, for example, the term liquid embolic may encompass a cast of the liquid embolic at a treatment site.

Although various embodiments are disclosed herein with specific reference to vaso-occlusive devices and treatments (e.g., to block blood flow in specific vessels, to treat cerebral aneurysms and other vascular abnormalities, etc.) for use by a physician in treating a subject, it shall be understood that this is for the sake of clear and efficient description. Thus, the catheters, systems, and methods of the present disclosure shall be understood to be useful in other types of medical treatments, unless a contrary intent is explicitly indicated or made clear from the context. As an example, unless otherwise specified or made clear from the context, the catheters, systems, and devices described herein may be used by a physician for treatment of any size defect and/or to treat any indication of a subject, as desired or required.

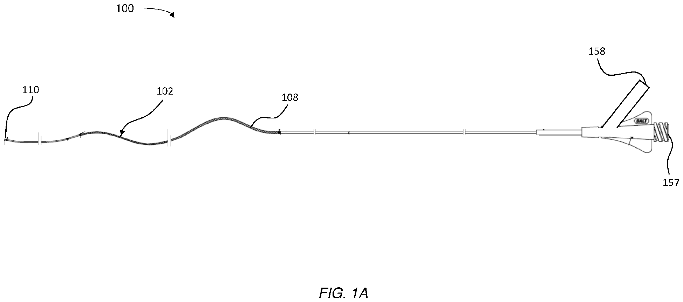

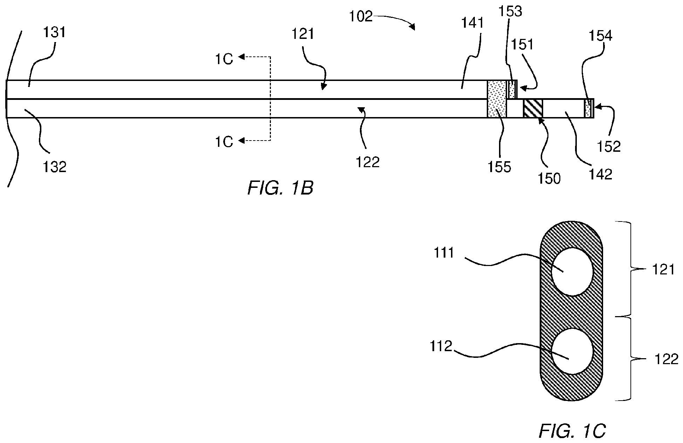

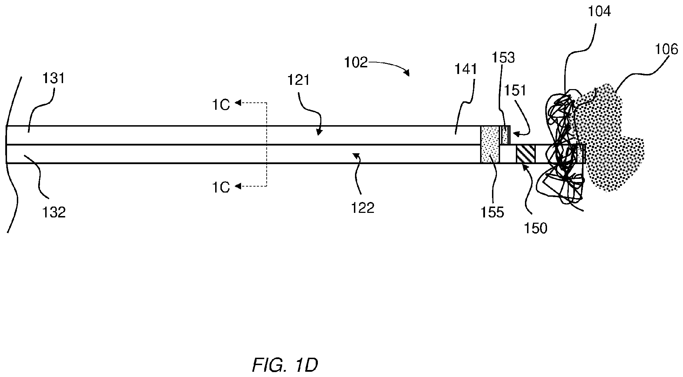

Referring now to FIGS. 1A and 1B, a system 100 for embolization of an anatomic vessel of a subject may include a catheter 102, a coil 104, and a liquid embolic 106 (the coil 104 and the liquid embolic 106 shown deployed at a treatment site in FIG. 1B). The catheter 102 may have a proximal region 108 and a distal region 110, with the catheter 102 defining a first lumen 111 and a second lumen 112 from the proximal region 108 to the distal region 110. In use, the coil 104 (e.g., Optima coils available from Balt USA, LLC, of Irvine, CA) may be deliverable to a treatment site of a subject via the first lumen 111, and the liquid embolic 106 may be deliverable to the treatment site of the subject via the second lumen 112. Upon injection to the treatment site, the liquid embolic 106 may be curable and, thus, may solidify on at least one section of the distal region 110 defining the second lumen 112, and this section of the distal region 110 may be detachable from the proximal region 108. That is, the distal region 110 may remain at the treatment site in instances in which a portion of the distal region 110 defining the second lumen 112 becomes entrapped in a cast of the liquid embolic 106 as the liquid embolic 106 solidifies. As an example, the liquid embolic 106 may have a composition including a water insoluble, biocompatible, non-biodegradable polymer, dissolved in a biocompatible solvent. Once the liquid embolic 106 is delivered out of the catheter 102 at the treatment site, the biocompatible solvent dissipates, leaving the non-biodegradable polymer to set up and solidify at the treatment site. Controlling delivery and placement of this type of the liquid embolic 106 may be useful, for example, for containing solidification to the treatment area while reducing the likelihood that solidification may occur in other areas in which it may be desirable to maintain blood flow. As an example, the liquid embolic 106 may include dimethyl sulfoxide (DMSO), such as the Squid liquid embolic, available from Balt USA, LLC, of Irvine, CA.

As an example, the catheter 102 may include a first tube 121 and a second tube 122. The first tube 121 may have a first proximal portion 131, a first distal portion 141, and the first lumen 111 may be defined therebetween. The first distal portion 141 may define a first orifice 151 in fluid communication with the first lumen 111. The second tube 122 may have a second proximal portion 132, a second distal portion 142, and a detachment zone 150 therebetween. The second tube 122 may define the second lumen 112 from the second proximal portion 132 to the second distal portion 142, with the second distal portion 142 defining a second orifice 152 in fluid communication with the second lumen 112. The first tube 121 may be coupled to the second tube 122 from second proximal portion 132 to at least the detachment zone 150 of the second tube 122. As described in greater detail below, the second distal portion 142 of the second tube 122 may be detachable from the second proximal portion 132 along the detachment zone 150. As compared to treating aneurysms using interventional devices delivered using separate catheters, the catheter 102 may facilitate simultaneous or nearly simultaneous delivery of the liquid embolic 106 through the second lumen 112 along with one or more other therapeutic devices through the first lumen 111. For example, the detachment zone 150 of the second tube 122 may facilitate leaving the second distal portion 142 of the second tube 122 at the treatment site in instances in which the second distal portion 142 of the second tube 122 becomes entrapped in a cast of the liquid embolic 106, as the liquid embolic 106 rapidly solidifies upon delivery to the treatment site. As a more specific example, the detachment zone 150 of the second tube 122 may facilitate decoupling the second distal portion 142 of the second tube 122 from at least a portion of the first tube 121 upon detachment of the second distal portion 142 from the second proximal portion 132 along the detachment zone 150 (e.g., when the second distal portion 142 is plugged in an embolic cast) such that the remainder of the catheter 102 may be removable from the vasculature.

In general, the second distal portion 142 may be detachable from the second proximal portion 132 of the second tube 122 at the detachment zone 150 according to application of a predetermined magnitude and direction of force that may be deliberately exerted at the detachment zone 150 by a physician manipulating the catheter 102 during treatment of a subject. That is, separation of the second distal portion 142 from the second proximal portion 132 of the second tube 122 may be generally achievable using only mechanical force. Thus, as compared to separation of a portion of a catheter using heat or other non-mechanical techniques, the detachment zone 150 of the catheter 102 may facilitate cost-effective and reliable separation.

In certain implementations, the second distal portion 142 of the second tube 122 may be detachable from the second proximal portion 132 at the detachment zone 150 in response to an axial stress exerted along the second tube 122. As an example, the axial stress under which separation may occur at the detachment zone 150 may be less than axial failure stress at any other axial position along the catheter 102 to reduce the likelihood of unintended separation of one or more portions of the catheter 102 as axial stress is applied to the catheter 102 to achieve separation at the detachment zone 150. For example, the axial stress that causes separation at the detachment zone 150 may be less than a first failure stress of the first tube 121 coupled to the second tube 122, as may be useful for reducing the likelihood that attempts to cause separation at the detachment zone 150 inadvertently causes separation of the coupling of the first tube 121 from the second tube 122 proximal to the detachment zone 150. Further, or instead, the axial stress that causes separation at the detachment zone 150 may be less than a second failure stress at any other axial position along the second tube 122 (e.g., less than failure stress of the second distal portion 142 of the second tube 122), as may be useful for reducing the likelihood of inadvertent separation along a portion of the second tube 122 away from the detachment zone 150.

In some instances, the detachment zone 150 may be an area of weakness in continuous material defining the second lumen 112 of the second tube 122. In some instances, the area of weakness may be formed as one or more structural features (e.g., jacketing) of the second tube 122. Further, or instead, the area of weakness at the detachment zone 150 may be attributable to a change in one more material properties at the detachment zone 150, relative to the same one or more material properties along portions of the second tube 122 away from the detachment zone 150. For example, the second tube 122 may be most brittle along the detachment zone 150 than along any other portion of the second tube 122. As a specific example, composition of the second tube 122 along the detachment zone 150 may differ from composition along any other portion of the second tube 122 such that the detachment zone 150 is the most brittle portion of the second tube 122. In certain instances, the second tube 122 may be formed of one or more polymers with tungsten, barium sulfate, or a combination thereof at the detachment zone 150 such that the detachment zone 150 is the most brittle portion of the second tube 122.

In certain implementations, the first distal portion 141 of the first tube 121 and the second distal portion 142 of the second tube 122 may be split from one another distal to the detachment zone 150. Stated differently, the first tube 121 and the second tube 122 may be fixed relative to one another (e.g., with jacketing along the first tube 121 and the second tube 122) from the first proximal portion 131 of the first tube 121 to the detachment zone 150 of the second tube 122, and the first tube 121 and the second tube 122 may be split from one another distal to the detachment zone 150. This may facilitate introducing the first distal portion 141 of the first tube 121 and the second distal portion142 of the second tube 122 together from an insertion site, through tortuous anatomy of the subject, to a treatment site while also facilitating relative movement of the first distal portion 141 and the second distal portion 142 at the treatment site for spatial separation of delivery of treatment at the treatment site. As an example, in instances in which the coil 104 is delivered to the treatment site via the first distal portion 141 and the liquid embolic 106 is delivered to the treatment site via the second distal portion 142, a split between the first distal portion 141 and the second distal portion 142 may reduce the likelihood of the liquid embolic 106 trapping the first distal portion 141.

Further, or instead, at least the first lumen 111 of the catheter 102 may remain operable following detachment of the second distal portion 142 from the second proximal portion 132 along the detachment zone 150. The continued operability of the first lumen 111 may be useful for carrying out one or more aspects of a treatment as the catheter 102 is retracted proximally following detachment of the second distal portion 142. For example, as the catheter 102 is retracted proximally, one or more instances of the coil 104 may be deployed through the first lumen 111. This may save time compared to deploying additional coils by inserting a second catheter.

In general, the first tube 121 and the second tube 122 may each have overall lengths based on the treatment to be carried out using the catheter 102. Specifically, the first tube 121 and the second tube 122 may each have an overall length that extends at least from an insertion point in the subject to a treatment site within vasculature of the subject. It shall be appreciated, therefore, that the first tube 121 and the second tube 122 may each have the same overall length in some instances, which may be useful for carrying out certain types of treatments. However, in certain instances, the second distal portion 142 of the second tube 122 may extend distally (e.g., by greater than about 5 mm and less than about 75 mm) beyond the first distal portion 141 of the first tube 121. This may be useful for deploying the coil 104 and the liquid embolic 106 simultaneously at the treatment site while achieving relative positioning in which the coil 104 is proximal to the liquid embolic 106 at the treatment site which, as described in greater detail below, may facilitate rapid and effective execution of certain types of treatment.

In general, the first lumen 111 and the second lumen 112 may each have respective cross-sectional areas through which one or more devices and/or treatments may be delivered to the treatment site according to the treatment to be carried out by the physician. That is, the first lumen 111 may have a first cross-sectional area (e.g., a circular cross-sectional area) from the first proximal portion 131 to the first orifice 151, and the second lumen 112 may have a second cross-sectional area (e.g. circular cross-sectional area) from the second proximal portion 132 to the second orifice 152. As an example, the first lumen 111 and the second lumen 112 may each have a respective maximum diameter greater than 0.5 mm and less than 0.5 mm, as may be useful for delivering devices and/or treatments at a treatment site while being navigable along tortuous vasculature (e.g., vasculature encountered in neurovascular treatments).

As an example, the first lumen 111 may have a constant cross-sectional area from the first proximal portion 131 to the first orifice 151, and the second lumen 112 may have a constant cross-sectional area from the second proximal portion 132 to the second orifice 152. Continuing with this example, constant first cross-sectional area and the constant cross-sectional area may be the same (e.g., within ± 10 percent of one another) to facilitate accommodating similar guidewires (e.g., 0.36 mm or 0.41 mm guidewires). Alternatively, one of the first lumen 111 or the second lumen 112 may have a larger cross-sectional area than the other to accommodate a 0.61 mm guidewire.

In certain implementations, one or more of the first lumen 111 or the second lumen 112 may be coated to facilitate delivery of one or more types of devices and/or treatments to the treatment site. For example, the catheter 102 may be hydrophobic along one or both of the first lumen 111 or the second lumen 112. As compared to a lumen without this property, hydrophobicity may reduce friction force that must be overcome to move components through the first lumen 111 and/or the second lumen 112, as the case may be, while providing good resistance to shedding that may unintentionally introduce material into the body of the subject.

In general, the first tube 121 and the second tube 122 may be formed of any one or more materials that are biocompatible and compatible with one another while satisfying the overall strength and flexibility requirements of the catheter 102 for carrying out a specific type of treatment. Thus, for example, the first tube 121 and the second tube 122 may each be at least partially formed of one or more polymers. As a specific example, the first tube 121 and the second tube 122 may each be at least partially formed of a respective polymer having Shore D hardness greater than 15D and less than 100D. Further, or instead, the first tube 121 and the second tube may each be formed of the same material, as may be useful for manufacturability of the catheter 102.

In some instances, at least a portion of each of the first tube 121 and the second tube 122 may include shielding in the one or more polymers proximal to the detachment zone 150. That is, shielding may be used to impart strength to the catheter 102 to hold the first tube 121 and the second tube 122 together while also being only in the portion of the catheter 102 that is retractable from the treatment site. Thus, the second distal portion 142 that is detachable to remain at the treatment site may be free of shielding. Further, or instead, the placement of shielding only proximal to the detachment zone 150 may facilitate forming a split between the first distal portion 141 of the first tube 121 and the second distal portion 142 of the second tube 122 distal to the detachment zone 150.

In certain implementations, the first tube 121 and the second tube 122 may each be directly coupled to one another, as may be useful for forming the catheter 102 with a small outer dimensional profile. For example, the first tube 121 and the second tube 122 may be directly coupled to one another from the second proximal portion 132 to at least the detachment zone 150 of the second tube 122. In this context, welding shall be understood to be an example of direct coupling between the first tube 121 and the second tube 122. Thus, in certain instances, the first tube 121 and the second tube 122 may be manufactured independently (e.g., to facilitate controlling different physical properties between the first tube 121 and the second tube 122), and the first tube 121 and the second tube 122 may be welded to one another at least from the second proximal portion 132 to at least the detachment zone 150 of the second tube 122 to form a shaft having an outer circumference.

In certain instances, the first tube 121 and the second tube 122 may collectively form a curvilinear outer circumference of the catheter 102. The curvilinear outer circumference of the catheter 102 may be at least at the detachment zone 150 and, in some implementations, may be constant at least from the first proximal portion 131 to the second proximal portion 132 to the detachment zone 150. The curvilinear outer circumference formed by the first tube 121 and the second tube 122 at least at the detachment zone 150 may have a major dimension and a minor dimension, with the major dimension greater than the minor dimension to facilitate imparting a preferential bending direction of the catheter 102 to facilitate navigability of the catheter 102 through tortuous vasculature. Examples of curvilinear outer circumferences having major dimensions greater than respective outer dimensions include oval-shaped and stadium-shaped, which are shapes that may be reliably manufactured with close dimensional tolerances.

The catheter 102 may, in some instances, include a first distal radiopaque marker 153 and a second distal radiopaque marker 154 to facilitate visualization of certain features of the first distal portion 141 and the second distal portion 142, respectively, as the catheter 102 is moved to and from the treatment site and while treatment is carried out at the treatment site. As an example, the first distal radiopaque marker 153 may be adjacent to the first orifice 151 of the first tube 121 and, further or instead, and the second distal radiopaque marker 154 may be adjacent to the second orifice 152 of the second tube 122. In this context, adjacency of the first distal radiopaque marker 153 to the first orifice 151 and adjacency of the second distal radiopaque marker 154 to the second orifice 152 shall be understood to include placement as close to the respective orifice as possible to provide accurate location of the respective orifice while also being securely supported on a respective one of the first tube 121 or the second tube 122, as the case may be. Thus, in this example, the first distal radiopaque marker 153 and the second distal radiopaque marker 154 may facilitate visualization of positioning of the first orifice 151 and the second orifice 152, which may be useful for accurate placement of the coil 104 and/or the liquid embolic 106 according to any one more of the various, different treatments described herein.

The catheter 102 may additionally, or alternatively, include a proximal radiopaque marker 155 proximal and adjacent to the detachment zone 150 of the second tube 122. The proximal radiopaque marker 155 may facilitate visualization of the detachment zone 150 (e.g., relative to each of the first orifice 151 and the second orifice 152) during treatment. In this context, adjacency of the proximal radiopaque marker 155 to the detachment zone 150 of the second tube 122 may include positioning that is as close to the detachment zone 150 as possible without interfering with detachment of the second distal portion 142 at the detachment zone 150 and remaining securely and completely coupled to at least the second tube 122 before and after detachment of the second distal portion 142.

In general, the catheter 102 may provide a physician with access for introduction of any one or more devices, treatments, or a combination thereof into the first tube 121 and the second tube 122 for delivery to the first proximal portion 131 of the first tube 121 and the second proximal portion 132 of the second tube 122, respectively, at the treatment site. Thus, for example, the first proximal portion of the first tube may define a first access port 157 in fluid communication with the first lumen 111 such that devices and/or treatments introduced into the first access port 157 may be delivered to the treatment site via the first distal portion 141 of the first tube 121. Further, or instead, the second proximal portion 132 of the second tube 122 may define a second access port 158 in fluid communication with the second lumen 112 such that devices and/or treatments introduced into the second access port 158 may be delivered to the treatment site via the second distal portion 142 of the second tube 122. To facilitate the use of the first lumen 111 and the second lumen 112 independently of one another for the delivery of devices and/or treatments, it shall be appreciated that the first lumen 111 and the second lumen 112 may be fluidically isolated from one another.

Having described certain structural aspects of the system 100 including the catheter 102, attention is now directed to aspects of use of the system 100 for treatment of a subject.

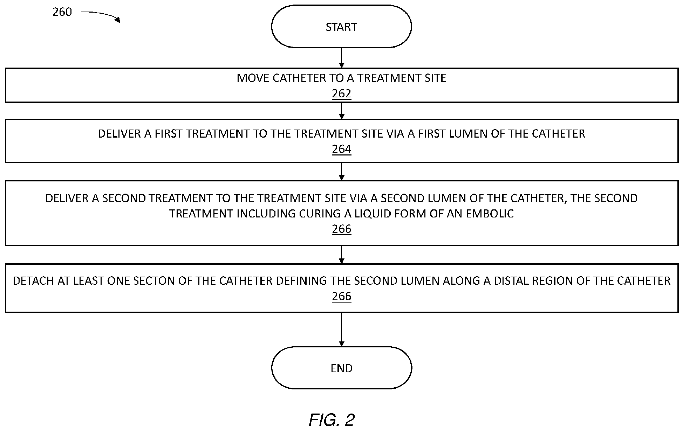

FIG 2 is a flowchart of an exemplary method 260 of embolization of an anatomic vessel. Unless otherwise specified or made clear from the context, it shall be understood that any one or more of the various different aspects of the exemplary method 260 may be carried out using the system 100 (FIG. 1A) including the catheter 102 (FIG. 1A).

As shown in step 262, the exemplary method 260 may include moving a catheter to a treatment site.

As shown in step 264, the exemplary method 260 may include delivering a first treatment to the treatment site via a first lumen defined from a proximal region to a distal region of the catheter. For example, the first treatment may include delivering a coil to the treatment site via the first lumen.

As shown in step 266, the exemplary method 260 may include delivering a second treatment to the treatment site via a second lumen defined from the proximal region to the distal region of the catheter, the second treatment including curing a liquid embolic (e.g., a liquid embolic including dimethyl sulfoxide (DMSO)). In certain instances, the coil may be delivered proximal to the liquid embolic at the treatment site. For example, the coil may be delivered to the treatment site with the liquid embolic solidified, or at least solidifying, at the treatment site such that the coil and the solidified liquid embolic collectively form a plug. In certain instances, the coil may be cast in the solidified liquid embolic. However, in some instances, the coil may be released and the direction of blood flow at the treatment site may push the coil against the solidified liquid embolic to form the plug. Thus, the coil and the liquid embolic may be delivered to the treatment site to carry out "pressure cooker embolization" using only a single catheter, which may reduce the time and complexity of the procedure carried out using multiple catheters.

Further, or instead, delivering the coil to the treatment site via the first lumen may include retracting the coil into the first lumen with the liquid embolic solidified at the treatment site. For example, the coil may be delivered to stop the flow while the liquid embolic is being delivered, and the coil may be retracted once the liquid embolic is delivered to the treatment site. Continuing with this example, as the catheter is retracted from the treatment site, the previously retracted instance of the coil may be deployed for blockage along another portion of the vasculature of the subject, thus reducing the need for using a second catheter. Further, or instead, retractability of the coil may be used to repositioning the coil at the treatment site being blocked by the solidification of the liquid embolic. As compared to treatment requiring introducing another coil for placement, it shall be appreciated that the retractability of the coil into the first lumen may reduce treatment time.

As shown in step 208, the exemplary method 260 may include, with the liquid embolic cured thereon, detaching at least one section of the distal region defining the second lumen.

While certain aspects of systems, catheters, and methods have been described herein, it shall be appreciated that other aspects are additionally or alternatively possible.

For example, while the detachment zone has been described as an area of weakness along a continuous material of a catheter, other types of detachment zones are additionally possible. For example, the detachment zone of any one or more of the catheters described herein may alternatively be an area of weakness resulting from a discontinuity in material along the second tube. For example, the second distal portion of the second tube may be releasably attached to the second proximal portion at the detachment zone via an interference fit that may be decoupled by axial stress on the second tube.

As another example, while catheters have been described as having a first tube and a second tube of different lengths (with the second tube extending distal to the first tube), other relative lengths of the first tube and the second tube are additionally or alternatively possible. For example, the first tube and the second tube may each have the same axial length such that a first distal portion of the first tube is detachable with detachment of a second distal portion of the second tube at the detachment zone.

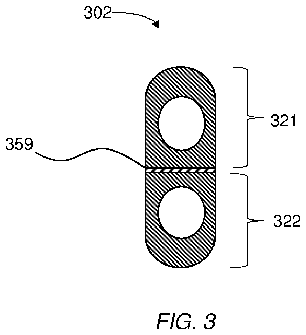

As yet another example, while catheters have been described as having a first tube and a second tube directly attached to each other, it shall be appreciated that other types of connection of the first tube and the second tube are additionally or alternatively possible. For example, referring now to FIG. 3, a catheter 302 may include a first tube 321, a second tube 322, and an intermediate member 359. For the sake of clear and efficient description, elements with 300-series numbers having the same last two digits as elements with 100-series numbers in this disclosure shall be understood to be analogous to or interchangeable with elements with one another, unless otherwise explicitly made clear from the context. Therefore, these elements are not described separately from one another, except to note differences or to emphasize certain features. Thus, for example, the catheter 302 shall be understood to be analogous to or interchangeable with the catheter 102 (FIG. 1A).

The first tube 321 and the second tube 322 may be coupled to one another from a second proximal portion to at least a detachment zone via the intermediate member 359. This may be useful as an additional degree of freedom in imparting physical properties to the catheter 302, as compared to directly coupling a first tube and a second tube to one another. For example, Shore D hardness of the intermediate member 359 may be different from respective Shore D hardness of each of the first tube 321 and the second tube 322.

The method steps of the implementations described herein are intended to include any suitable method of causing such method steps to be performed, consistent with the patentability of the following claims, unless a different meaning is expressly provided or otherwise clear from the context. So, for example performing the step of X includes any suitable method for causing another party such as a remote user, a remote processing resource (e.g., a server or cloud computer) or a machine to perform the step of X. Similarly, performing steps X, Y and Z may include any method of directing or controlling any combination of such other individuals or resources to perform steps X, Y and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity, and need not be located within a particular jurisdiction.

It will be appreciated that the methods and systems described above are set forth by way of example and not of limitation. Numerous variations, additions, omissions, and other modifications will be apparent to one of ordinary skill in the art. Absent an explicit indication to the contrary, the disclosed steps may be modified, supplemented, omitted, and/or re-ordered without departing from the scope of this disclosure. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the spirit and scope of this disclosure and are intended to form a part of the invention as defined by the following claims.

The following numbered clauses define examples of the present disclosure and form an additional part of the Summary section of the description.
1. A catheter for embolization of an anatomic vessel of a subject, the catheter comprising:
   a first tube having a first proximal portion, a first distal portion, and a first lumen defined therebetween, the first distal portion defining a first orifice in fluid communication with the first lumen; and
   a second tube having a second proximal portion, a second distal portion, and a detachment zone therebetween, the second tube defining a second lumen from the second proximal portion to the second distal portion, the second distal portion defining a second orifice in fluid communication with the second lumen, the first tube coupled to the second tube from the second proximal portion to at least the detachment zone, and the second distal portion of the second tube detachable from the second proximal portion along the detachment zone.
2. The catheter of any one of the preceding clauses, wherein the second distal portion of the second tube becomes decoupled from at least a portion of the first tube upon detachment of the second distal portion of the second tube from the second proximal portion along the detachment zone.
3. The catheter of any one of the preceding clauses, wherein the second distal portion of the second tube is detachable from the second proximal portion in response to an axial stress less than a first failure stress of the first tube coupled to the second tube and less than a second failure stress of the second distal portion of the second tube.
4. The catheter of any one of the preceding clauses, wherein at least the first lumen remains operable following detachment of the second distal portion from the second proximal portion along the detachment zone.
5. The catheter of any one of the preceding clauses, wherein the second distal portion of the second tube extends distally beyond the first distal portion of the first tube.
6. The catheter of any one of the preceding clauses, wherein the first distal portion of the first tube and the second distal portion of the second tube are split from one another distal to the detachment zone.
7. The catheter of any one of the preceding clauses, wherein, at least at the detachment zone, the first tube and the second tube collectively form a curvilinear outer circumference having a major dimension and a minor dimension, and the major dimension is greater than the minor dimension.
8. The catheter of any one of the preceding clauses wherein the first proximal portion of the first tube defines a first access port in fluid communication with the first lumen, the second proximal portion of the second tube defines a second access port in fluid communication with the second lumen, and the first lumen and the second lumen are each fluidically isolated from one another.
9. The catheter of any one of the preceding clauses, wherein the first lumen has a first cross-sectional area from the first proximal portion to the first orifice, and the second lumen has a second cross-sectional area from the second proximal portion to the second orifice.
10. The catheter of any one of the preceding clauses, wherein the first tube is hydrophobic along the first lumen, and the second tube is hydrophobic along the second lumen.
11. The catheter of any one of the preceding clauses, wherein the first tube and the second tube are each at least partially formed of one or more polymers.
12. The catheter of any one of the preceding clauses, further comprising a proximal radiopaque marker proximal and adjacent to the detachment zone of the second tube.
13. The catheter of any one of the preceding clauses, further comprising a first distal radiopaque marker and a second distal radiopaque marker, wherein the first distal radiopaque marker is adjacent to the first orifice of the first tube, and the second distal radiopaque marker is adjacent to the second orifice of the second tube.
14. The catheter of any one of the preceding clauses, wherein the first lumen and the second lumen each have a diameter greater than 0.05 mm and less than 0.5 mm.
15. The catheter of any one of the preceding clauses, wherein the first tube and the second tube are directly coupled to one another from the second proximal portion to at least the detachment zone.

## Claims

1. A catheter for embolization of an anatomic vessel of a subject, the catheter comprising:
a first tube having a first proximal portion, a first distal portion, and a first lumen defined therebetween, the first distal portion defining a first orifice in fluid communication with the first lumen; and
a second tube having a second proximal portion, a second distal portion, and a detachment zone therebetween, the second tube defining a second lumen from the second proximal portion to the second distal portion, the second distal portion defining a second orifice in fluid communication with the second lumen, the first tube coupled to the second tube from the second proximal portion to at least the detachment zone, and the second distal portion of the second tube detachable from the second proximal portion along the detachment zone.

2. The catheter of any one of the preceding claims, wherein the second distal portion of the second tube is decouplable from at least a portion of the first tube upon detachment of the second distal portion of the second tube from the second proximal portion along the detachment zone.

3. The catheter of any one of the preceding claims, wherein the second distal portion of the second tube is detachable from the second proximal portion in response to an axial stress less than a first failure stress of the first tube coupled to the second tube and less than a second failure stress of the second distal portion of the second tube.

4. The catheter of any one of the preceding claims, wherein the second tube is most brittle along the detachment zone than along any other portion of the second tube.

5. The catheter of claim 4, wherein composition of the second tube along the detachment zone differs from composition along any other portion of the second tube.

6. The catheter of any one of the preceding claims, wherein, at least at the detachment zone, the first tube and the second tube collectively form an oval-shaped or stadium shaped outer circumference.

7. The catheter of any one of the preceding claims wherein the first proximal portion of the first tube defines a first access port in fluid communication with the first lumen, the second proximal portion of the second tube defines a second access port in fluid communication with the second lumen, and the first lumen and the second lumen are each fluidically isolated from one another.

8. The catheter of any one of the preceding claims, wherein the first lumen has a first cross-sectional area from the first proximal portion to the first orifice, and the second lumen has a second cross-sectional area from the second proximal portion to the second orifice.

9. The catheter of any one of the preceding claims, wherein the first tube is hydrophobic along the first lumen, and the second tube is hydrophobic along the second lumen.

10. The catheter of claim 9, wherein the first distal portion of the first tube and the second distal portion of the second tube are each movable relative to one another distal to the detachment zone of the second tube.

11. The catheter of any one of the preceding claims, wherein the first tube and the second tube are each at least partially formed of one or more polymers, and at least a portion of each of the first tube and the second tube includes shielding in one or more polymers proximal to the detachment zone.

12. The catheter of any one of the preceding claims, wherein the first tube and the second tube are welded to one another from the second proximal portion to the detachment zone.

13. The catheter of any one of the preceding claims, wherein the second distal portion of the second tube at least partially extends distally beyond the first distal portion of the first tube.

14. The catheter of any one of the preceding claims, wherein the first distal portion of the first tube and the second distal portion of the second tube are each movable relative to one another distal to the detachment zone of the second tube.

15. The catheter of any one of the preceding claims, wherein the first lumen has a first cross-sectional area from the first proximal portion to the first orifice, and the second lumen has a second cross-sectional area from the second proximal portion to the second orifice, the first cross-sectional area is constant from the first proximal portion to the first orifice, and the second cross-sectional area is constant from the second proximal portion to the second orifice.
